# EUROPEAN PATENT APPLICATION

(11) **EP 4 644 895 A1**
(43) Date of publication of application: **05.11.2025**
(21) Application number: 25173289.7
(22) Date of filing: 29.04.2025
(51) Int. Cl.: G01N 33/00

(54) **ENVIRONMENTAL SENSOR ASSEMBLY**

(30) Priority: 03.05.2024 LU 507114
(71) Applicant: FLUSSO LIMITED, Cambridge, Cambridgeshire CB3 0QH (GB)
(72) Inventor: DIXON, Sean, Cambridge, CB3 0QH (GB); LEE, Cerdin, Cambridge, CB3 0QH (GB); GARDNER, Ethan Lance, Cambridge, CB3 0QH (GB); ALI, Syed Zeeshan, Cambridge, CB3 0QH (GB)
(74) Representative: Marks & Clerk LLP

(57) **Abstract**

An environmental sensor assembly comprising: a package; and an environmental sensor disposed in the package, the environmental sensor comprising a sensing region and a reference region; wherein the package comprises one or more inlets arranged symmetrically with respect to the sensing region and the reference region; and wherein the sensing region is in fluid communication with an external environment through the one or more inlets. A method of manufacturing an environmental sensor assembly is also described.

## Description

### TECHNICAL FIELD

The present disclosure relates to environmental sensor assemblies comprising packaged environmental sensors such as fluid sensors (e.g. gas sensors and/or including humidity sensors) or particle sensors.

### BACKGROUND

Environmental sensors, such as fluid sensors or gas sensors, are in widespread use in a number of applications due to low cost, small size, and ability to measure a large number of fluids (e.g. gases) and in large range of concentrations.

Environmental sensors may comprise a sensing region and a reference region and any circuitry ensuring the functioning of the sensor for the chosen application.

A sensor comprising a reference region may provide accurate results across a wide range of sensing conditions. The presence of a reference region, on the other hand, adds to the overall dimensions of the device.

Generally, an environmental sensor is provided inside a package which may protect the environmental sensor, and any other electrical component or connection, from the external environment, including any flow which may affect the accuracy of the environmental sensor reading. The combination of a environmental sensor and the package may be known as an environmental sensor assembly.

A stringent requirement for any environmental sensor is having a fast response time. For example, a sensor should detect a gas leak as soon as possible so that a system can counteract and prevent a potentially destructive outcome, like as an explosion, poisoning or some serious malfunctioning.

Any system's response time, however, is only partly dependent on the type and technology of sensor used within it. A large part of the response time is also determined by the time the gas (or other fluid) takes to reach the sensing area of the sensor. Sensors are generally located in a package and/or a casing. It is possible to speed up the transport of gas molecules to a sensor casing or package, for example, by providing a fan, a blower or by exploiting convection and buoyancy. Once the gas molecules reach an aperture in the sensor casing or package, though, the time necessary for those molecules to reach the sensing area of the sensor is dictated by gas diffusion.

For a sensor assembly having an environmental sensor comprising a sensing region and a reference region, it is desirable that the sensing region and the reference region are subject to the same kind of thermal environment and/or interaction which require careful design of the package.

For ease of integration in a wide range of systems and for a wide range of applications, it is desirable for a sensor assembly to provide a self-contained device of low encumbrance, so as to be integrated even in space constrained applications.

EP 2 952 886 B1 relates to a method for manufacturing a gas sensor package. US 8,916,408 B2 relates to a leadframe-based premolded package. US 9,708,174 B2 relates to a process for manufacturing a packaged device.

### SUMMARY

The present disclosure is directed to an environmental sensor assembly comprising a package, and an environmental sensor disposed in the package, which may address one or more of the above issues.

The environmental sensor comprises a sensing region (also referred to as a sensing portion), and a reference region (also referred to as a reference portion). The sensor assembly of the present disclosure provides fast and accurate sensing (e.g. of fluids such as gases) by providing a short diffusion path for target (e.g. gas) molecules to diffuse from outside a package inlet to the sensing region and by reducing any possible thermal imbalance between the sensing region and the reference region. Additionally, the present disclosure provides a self-contained and compact sensor assembly which is easy to integrate in a wide range of systems and for a wide range of applications.

The package may shield the sensor from unwanted contaminations or interferences while ensuring effective interaction with the environment for the sensor to function reliably.

Aspects and preferred features are set out in the accompanying claims.

Described herein is an environmental sensor assembly comprising a package, and an environmental sensor disposed in the package. The environmental sensor comprises a sensing region and a reference region. The package comprises one or more inlets arranged symmetrically with respect to the sensing region and the reference region. The sensing region is in fluid communication with an external environment through the one or more inlets.

Also described herein is a method of manufacturing an environmental sensor assembly, the method comprising disposing an environmental sensor in a package, the environmental sensor comprising a sensing region and a reference region, and forming one or more inlets in the package, the one or more inlets being arranged symmetrically with respect to the sensing region and the reference region, and such that the sensing region is in fluid communication with an external environment through the one or more inlets.

It will be understood that sensing region is where the interaction between a target fluid (e.g. gas) and the sensor takes place in order to produce a sensor reading. The reference region may be fluidically isolated from the external environment.

It is desirable that the sensing region and the reference region are exposed to same environmental conditions (e.g. the same external temperature). In this way, when differential sensor readings are collected, any effect of the environmental conditions is reduced or cancelled out. This allows to obtain more accurate sensor readings and allows to increase the sensitivity of the environmental sensor. The reference region may not to be in contact with the target fluid. The sensing region and the reference region are otherwise identical, in preferred implementations.

The package protects the environmental sensor from the external environment, especially from any foreign matter, dust and/or any flow which may affect the accuracy of the sensor readings. The package, therefore, provides a degree of isolation of the sensor from the external environment. On the other hand, the sensor is to be exposed to the external environment to provide meaningful readings and such exposure is mediated by the package by means of one or more inlets (e.g. apertures) which put the sensor in fluid communication with the external environment. It is desirable that the package allows the sensing region and the reference region to be influenced by the external environment in a fashion which is the closest possible.

The symmetric arrangement of the one or more inlets of the package, with respect to the sensing region and the reference region of the environmental sensor, may allow substantially homogeneous thermal interaction between both the sensing region and the reference region and the external environment. This allows accurate reduction or cancellation of external effects (e.g. a variation of the external temperature) on the environmental sensor readings.

The package may comprise a package substrate and/or a lid to enclose the environmental sensor. The environmental sensor may be located on a surface of the package substrate.

In some examples, the environmental sensor comprises a sensor substrate. The sensing region may comprise a sensing area of the sensor substrate, and the reference region may comprise a reference area of the sensor substrate. The sensor substrate may comprise a first main surface facing the package substrate and a second, opposite, main surface. The lid may be arranged, at least in part, opposite the second main surface of the sensor substrate. The sensing area and the reference area may be provided on the second main surface of the sensor substrate.

The package may be provided with a single inlet.

The package may comprise a plurality of inlets.

The package may be provided with an inlet opposite the sensing region and an inlet opposite the reference region.

The package may comprise one or more lateral, e.g. vertical, walls between the package substrate and the lid.

One or more lateral walls may be integral to the lid.

One or more lateral walls may be integral to the package substrate.

The package may be of cuboid shape or any other suitable shape.

The package may comprise any suitable material, such as a polymer, a ceramic, a metal, a composite, etc.

The package substrate may comprise a lead frame, a printed circuit board (PCB) and/or plastic.

The package may be provided with an internal wall arranged to maintain the reference region fluidically isolated from the rest of the package and, in turn, from the external environment. Preferably, the internal wall may comprise a material with high thermal conductivity so that the thermal interaction between the reference region and the external environment may be maintained as close as possible to the thermal interaction between the sensing region and the external environment.

In some examples, an internal wall may not be provided and the reference region may be fluidically isolated from the external environment thanks to a suitable coating or isolating material which does not allow the target fluid to interact with the reference area.

For easier manufacturability of the environmental sensor, the sensing area and the reference area may be provided on the second main surface of the gas sensor substrate.

The one or more inlets may be provided, at least in part, on the lid so as to be located, at least in part, opposite the second main surface of the sensor substrate. In examples where the sensing area and the reference area are provided on the second main surface of the sensor substrate, this may provide a close proximity of the sensing area to the external environment which may provide a short diffusion path of target molecules to the sensing area so that a sensor reading may be obtained quickly.

The one or more inlets may be provided, at least in part, on one or more vertical or lateral walls, so as to be in a non-overlapping relation with the second main surface of the sensor substrate. This may provide a higher level of protection of the sensing area (and/or sensing region) from any external matter, dust and/or flow; on the other hand this may extend the diffusion path from the one or more inlets to the sensing area area and, in turn the time required for the output of a sensing reading.

The environmental sensor assembly may comprise a cap defining a reference chamber, the reference region comprising the reference chamber, such that the reference region is fluidically isolated from the one or more inlets (i.e. from the package). Preferably, the cap comprises a material with high thermal conductivity (e.g. silicon or metal) so that the thermal interaction between the reference region and the external environment may be maintained as close as possible to the thermal interaction between the sensing region and the external environment.

The cap may define two separate chambers: a sensing chamber (which may enclose the sensing area), the sensing region comprising the sensing chamber, and a reference chamber (which may enclose the reference area), the reference region comprising the reference chamber. The sensing chamber may comprise one or more holes such that the sensing region is maintained in fluid communication with the one or more inlets (i.e. with the package). The reference chamber may be arranged to seal the reference region from the package.

The sensing chamber and the reference chamber may be of the same shape and/or size.

The package may be provided with a single inlet opposite a central region of the cap, for example opposite a central region of a symmetry axis of the cap. Alternatively, the package may be provided with a plurality of inlets arranged symmetrically with respect to an axis of symmetry of the cap, e.g. in a pattern symmetrical having a same symmetry axis, or symmetry center as the cap.

In some embodiments, the package may be provided with a set of one or more inlets facing the sensing region and a set of one or more inlets facing the reference region, the two sets of one or more inlets being arranged symmetrically in relation to the respective sensing or reference region. This kind of arrangement may allow a flow of fluid (e.g. gas) inside the package to quickly fill the package internal volume with fluid from the external environment, while such flow may not affect the first sensing region because the fluid may diffuse, rather than flow, through the hole(s) in the cap.

The reference region (which may include the reference chamber) may be under vacuum (e.g. at a pressure of or below0.1 kPa), or it may be filled with a fluid (e.g. a gas) or a gas mixture of controlled composition and pressure. For example, the reference chamber may be filled with air or nitrogen at atmospheric pressure (~100 kPa).

Preferably, the one or more holes of the sensing chamber (i.e. the one or more holes in the cap) are laterally displaced with respect to the one or more inlets of the package, e.g. the one or more holes may be in non-overlapping relation with the one or more inlets of the package. This may provide a higher level of protection of the sensing region, especially the sensing area, from the external environment by avoiding the formation of a direct or straight path from the outside atmosphere to the sensing region.

In some embodiments, one or more inlets and/or one or more holes may be provided with a filter. In some cases, an inlet provided with a filter may overlap one or more holes. In some examples one or more holes provided with a filter may be in overlapping relation with an inlet.

The sensing chamber may be provided with a single hole in the cap. The single hole may be as large as possible so that a high number of target fluid (e.g. gas) molecules are allowed to diffuse to the sensing region in the unit of time but not so large that the sensing region is not suitably protected from the external environment. Preferably, the sensing chamber may be provided with a plurality of holes of comparatively smaller dimensions so as to ensure good protection of the sensing region and his diffusion of target fluid molecules to the sensing region in the unit of time.

In some implementations, the environmental sensor comprises: a substrate comprising a sensing cavity within the sensing region and a reference cavity within the reference region; a dielectric layer disposed over the substrate, wherein the sensing cavity defines a sensing membrane in the dielectric layer, and wherein the reference cavity defines a reference membrane in the dielectric layer; and a first heating element on or within the sensing membrane, and a second heating element on or within the reference membrane.

The sensor substrate provided with a dielectric layer may also be defined as a sensor die. The surface of the sensor die having the dielectric layer may be defined as the second surface of the sensor die.

In some examples, the sensing membrane may correspond to the sensing area described herein, and the reference membrane may correspond to the reference area described herein. It is desirable that, when a cap with one or more holes in the sensing chamber is provided, the one or more holes are laterally displaced (e.g. in a non-overlapping relation) with respect to the sensing membrane. This enables more effective protection of the sensing membrane. It will be understood that the sensing chamber and the reference chamber may enclose an area larger than the respective membranes, or the respective sensing and reference areas.

The sensor substrate may comprise any suitable material. For example, the sensor substrate may comprise a semiconductor, such as silicon.

The dielectric layer may comprise any suitable material, for example silicon oxide, silicon nitride, aluminium oxide or a combination of these materials.

The heating elements may comprise platinum, tungsten, titanium, single crystal silicon, polysilicon, and/or another suitable material. The type of heating element, its shape and the material it is made of are not limited and any heating element known in the art may be used in the implementation of the sensors described herein.

In some examples, the package may comprise a first portion having a first thickness in a direction perpendicular to a surface of the package facing the sensor, and a second portion having a second thickness in a direction perpendicular to the surface of the package facing the sensor, wherein the first thickness is different from the second thickness. For example, the package may be structured to match, at least in part, the shape of the sensor so that the internal dead volume of the package is reduced or minimized. This may contribute to improve the responsiveness of the sensor.

Portions of different thickness may be provided in one or more lateral walls and/or in the lid.

In some embodiments, the thickest portion of one or more lateral walls may be adjacent to the sensor so as to have, at least in part, the same shape as the volume occupied by the sensor and to be separated from the sensor only by a minimum offset, or clearance, as required for manufacturing and positioning of the sensor. In other words, part of the walls may follow part of the perimeter of the sensor.

A distance between the environmental sensor and a wall of the one or more walls closest to the environmental sensor may be less than 100 µm. More preferably, a distance between the environmental sensor and a wall of the one or more walls may be less than 50 µm.

In some instances, a surface of the lid facing the sensor may have a shape matching, at least in part, a shape of the sensor. For example, a thickness of a portion of the lid facing the sensor may be different from a thickness of a portion of the lid not facing the sensor, e.g., facing a region of the package provided with connection pads or other features, or a region of the package which is empty.

In an example, a distance between the lid and the second surface of the sensor die, or the external surface of the cap, when present, may be 50 µm.

It will be understood that the size and number of the inlets and a thickness of a portion of the package provided with one or more inlets, which in turn defines the size of the inlet(s) in a direction through the package, may be optimized in a way so that the internal volume of the package is conveniently reduced and that the diffusion of target fluid molecules through the inlet(s) is not too restricted.

For example, a portion of the package comprising an inlet may have a lower thickness than a portion of the package removed from an inlet.

The environmental sensor may be a thermal conductivity sensor (e.g. a thermal conductivity gas sensor), wherein the sensor responds to changes in the thermal conductivity of fluid (e.g. gas) around it due to change in fluid composition. For such a sensor type, preferably, the reference area may be isolated from the external environment while the sensing area may be exposed to the environment.

The environmental sensor may be a catalytic sensor (e.g. a catalytic gas sensor) comprising, in the sensing area, a heating element and a catalyst, where, at high temperature, the catalyst catalyzes the combustion of a target fluid (e.g. gas) causing an increase in temperature that can be measured either by the heating element or by a separate temperature sensor. In some examples, both the sensing area and the reference area may have a catalyst and the reference area may be isolated from the external environment while the sensing area may be exposed to the external environment. In other examples, both the sensing area and the reference area may be exposed to the external environment, but the reference area may not have a catalyst, or the reference area may have a catalyst which is coated by some other material to prevent the interaction between the catalyst and the surrounding fluid.

The environmental sensor may be a resistive or capacitive sensor comprising, in the sensing area, a heating element and a sensing material whose resistance or capacitance changes in the presence of a target fluid (e.g. gas). In some examples, both reference and sensing areas may be provided with the same sensing material and the reference area may be isolated from the external environment. In other examples both areas may not be isolated from the external environment. In this case, the reference area may not be provided with a sensing material, or the sensing material may be coated to prevent interaction between the sensing material and the surrounding fluid.

The environmental sensor assembly may further comprise a circuit chip, such as an application-specific integrated circuit (ASIC), located inside the package, to control and operate the sensor.

The circuit chip may be located on a surface of the package substrate.

The circuit chip may have a thickness in a direction perpendicular to the surface of the package substrate lower than a thickness of the environmental sensor in the same direction.

In some examples the thickness of the circuit chip is lower than the thickness of the environmental sensor.

When the circuit chip is provided inside the package, the package may be structured to match, at least in part, the shape of the environmental sensor and the shape of the circuit chip. In some embodiments, a surface of the lid facing the environmental sensor and the circuit chip has a shape matching, at least in part, a shape of the environmental sensor and the circuit chip. For example, the thickness of a portion of the lid facing the environmental sensor may be different from (e.g. lower than) a thickness of a region of the lid facing the circuit chip.

### BRIEF DESCRIPTION OF THE DRAWINGS

The present invention will now be described, by way of example only, with reference to the following drawings:
Figure 1: cross-section of an example of an environmental sensor assembly according to the present disclosure;
Figure 2: cross-section of an example of an environmental assembly having sensing and reference regions comprising catalytic or oxide layers according to the present disclosure;
Figure 3: cross-section of an example of an environmental sensor assembly comprising an environmental sensor comprising a cap according to the present disclosure;
Figure 4: cross-section of an example of an environmental sensor assembly comprising a sensing cavity and a reference cavity defined in the sensor substrate;
Figure 5A: top view of an example of an environmental sensor according to the present disclosure;
Figure 5B: side view of the environmental sensor illustrated in Figure 5A;
Figure 6A: top view of an example of an environmental sensor assembly comprising an application-specific integrated circuit (ASIC) according to the present disclosure;
Figure 6B: side cross-section of the environmental sensor assembly illustrated in Figure 6A;
Figure 7A: top view of an example of an environmental sensor assembly comprising an inlet that is elongated in one direction according to the present disclosure;
Figures 7B to 7D: top view of examples of environmental sensor assemblies comprising pluralities of inlets according to the present disclosure; and
Figure 8: example of a method of manufacturing an environmental sensor assembly according to the present disclosure.

### DETAILED DESCRIPTION

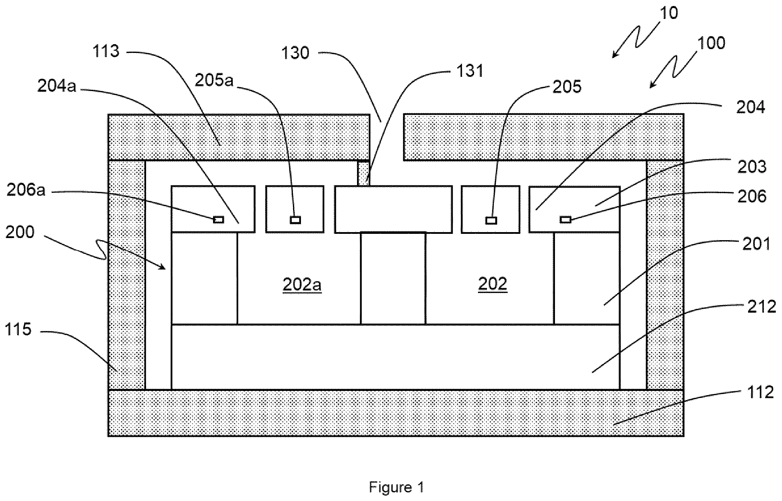

Figure 1 shows a cross-section of an example of an environmental sensor assembly 10 according to the present disclosure.

The environmental sensor assembly 10 comprises a package 100 and an environmental sensor 200 (also referred to herein as a "sensor"). The package 100 has a lid 113, a package substrate 112, lateral walls 115 and an inlet 130. The sensor 200 comprises a sensor substrate 201, a bottom substrate 212 and a dielectric layer 203. The sensor substrate 201 has a first surface facing the package substrate and a second, opposite surface. The dielectric layer 203 is provided on the second surface of the sensor substrate. The sensor substrate 201 is further provided with a sensing cavity 202 and a reference cavity 202a which are closed at the bottom by the bottom substrate 212. The sensing cavity 202 and the reference cavity 202a define, in the dielectric layer, respectively, a first or sensing membrane 204 and a second or reference membrane 204a, each membrane provided with a respective heating element 205, 205a. Next to each membrane are respective temperature sensors 206, 206a. As it can be seen, the sensor 200 has two symmetrical regions, the region on the righthand side of Figure 1 being a sensing region and the region on the lefthand side of Figure 1 being a reference region. The area of the first membrane 204 provided with the heating element 205 is the sensing area. The area of the second membrane 204a provided with the heating element 205a is the reference area.

The package 100 is further provided with an internal wall 131 which fluidically isolates the reference region from the external environment, while the sensing region is fluidically connected with the external environment through the inlet 130. The sensor 200 is operated and controlled by a controller or readout circuit (not shown). In this case, the heating element 205 may function as a heating and sensing element and interact with a target gas to determine a sensor output. The sensor output may be based on a differential signal obtained using a signal returned by the heating element 205 and a signal returned by the heating element 205a, which is not exposed to the target gas. The heating elements 205 and 205a are otherwise exposed to the same, or as close as possible, environmental conditions. In some cases, the membranes 204 and 204a may be provided with separate sensing elements (not shown) in thermal contact with the respective heating elements 205 and 205a. The temperature sensors 206 and 206a are arranged outside of the membranes 204 and 204a and are used to measure the ambient temperature of the sensor which may be affected by the external temperature, i.e. the temperature of the environment outside the sensor assembly 10.

The inlet 130 is arranged in a position which is symmetrical with respect to the sensing region and the reference region. The package 100 may be provided with more than one inlet 130, as long as the inlets for a pattern which is symmetrical with respect to the sensing region and the reference region, so that both regions experience the same, or as close as possible, environmental conditions, e.g. external temperature variations.

Figure 1 shows the inlet 130 being provided on the lid of the package 100 but this is in no way limiting and one or more inlets 130 may be provided, in addition or instead, on one or more lateral walls 115, as long as they form a symmetrical arrangement with respect to the sensing region and the reference region. The inlet 130 is in non-overlapping relation with either the sensing area or the reference area.

It will be understood that the internal wall 131 may be arranged differently, as long as it isolates fluidically the reference region from the external environment.

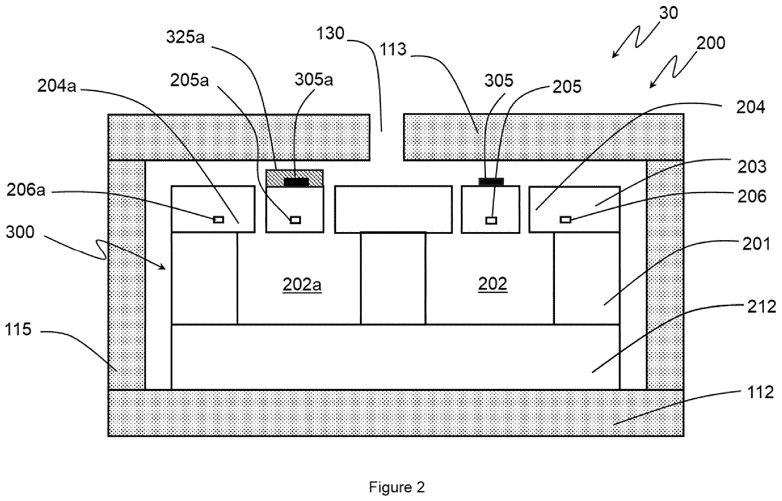

Figure 2 shows a cross-section of an example of the environmental sensor assembly 30 according to the present disclosure. The environmental sensor 300 of the environmental sensor assembly 30 of Figure 2, is similar to the environmental sensor 200 of Figure 1 except for the sensing area and the reference area which, in the environmental sensor 300 of Figure 2 are further provided with respective catalytic or oxide layers 305, 305a. In this case, the interaction between the sensor and a target fluid takes place through an interaction of the target fluid with the layer 305. The layer 305a is covered by a coating 325a which prevents the layer 305a from interacting with the external environment. In this example, the internal wall 131 shown in Figure 1, which fluidically isolates the reference area from the external environment may be dispensed with, as the reference area will be fluidically isolated from the external environment by the coating 325a. Furthermore, the bottom substrate 212 although shown is also not necessary in this configuration.

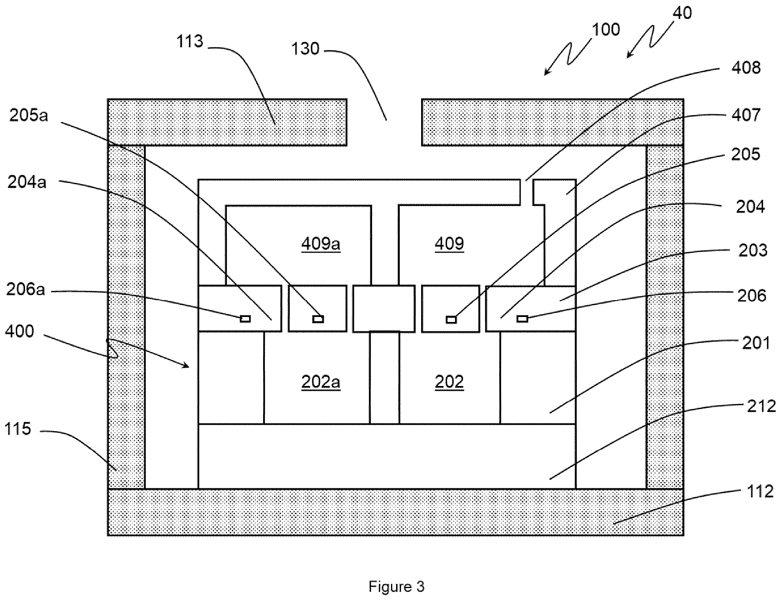

Figure 3 shows the cross-section of a further example of an environmental sensor assembly 40 according to the present disclosure. The environmental sensor assembly 40 of Figure 3 comprises an environmental sensor 400, which is similar to the environmental sensor 200 of Figure 1. With respect to the environmental sensor 200 of Figure 1, the environmental sensor 400 of Figure 3 further comprises a cap 407 provided over the second surface of the sensor substrate 201. The cap 407 defines a sensing chamber 409 and a reference chamber 409a. The reference chamber 409a fluidically isolates the reference region (comprising the reference area) from the external environment. The sensing chamber 409 is provided with a hole 408 which fluidically connects the sensing region (comprising the sensing area) to the internal volume of the package 100, and, in turn, to the external environment. In this example the inlet 130 and the hole 408 are in non-overlapping relation.

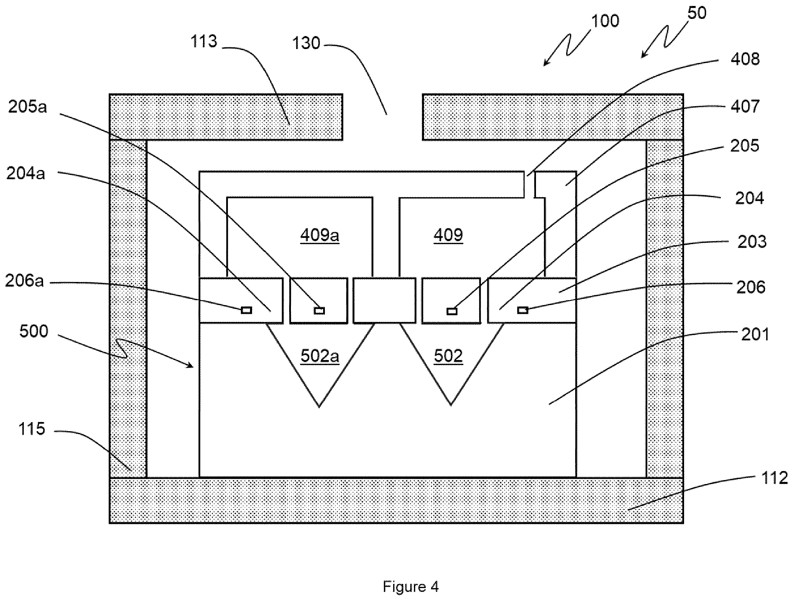

Figure 4 shows a cross-section of an environmental sensor assembly 50 similar to the environmental sensor assembly 40 of Figure 3. Figure 4 shows an environmental sensor 500 which does not comprise a bottom substrate as the sensing cavity 502 and the reference cavity 502a are completely defined in the sensor substrate 201.

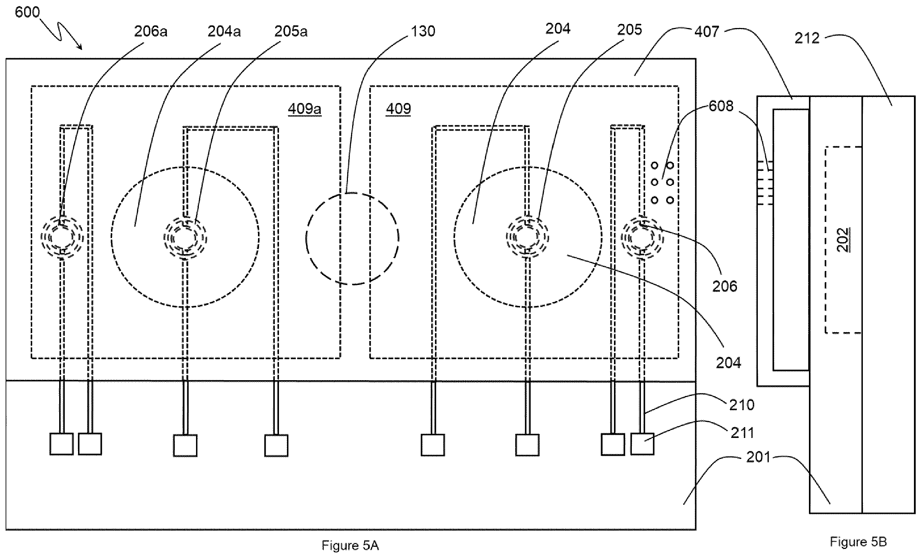

Figure 5A depicts a top view of an environmental sensor 600 similar to the environmental sensor of Figure 3 or to the environmental sensor of Figure 4. The environmental sensor 600 is shown with the cap 407 on, therefore the features that are underneath the cap 407 are shown in broken lines. The environmental sensor 600 is shown without the package. Figure 5B is a side view of sensor 600. Heating elements 205 and 205a and temperature sensors 206 and 206a are shown as microplates but this is not limiting. Elements 205, 205a, 206 and 206a are connected, through electrical traces 210, to pads 211 which are located on the second surface of the sensor substrate 201. The cap 407 is narrower than the sensor substrate 201 so that the pads 211 are exposed. The membranes 204 and 204a are shown having a circular shape but any other shape can be chosen instead. In this example, cap 407 is provided with a plurality of holes 608. The holes 608 do not overlap with the sensing area, nor with the sensing membrane 204. As it can be seen, the projection of the inlet 130 on the cap 407 is depicted to show that the inlet 130 of the package is arranged symmetrically with respect to the sensing region and the reference region of the sensor 600 and in non-overlapping relation with the holes 608 of the cap 407.

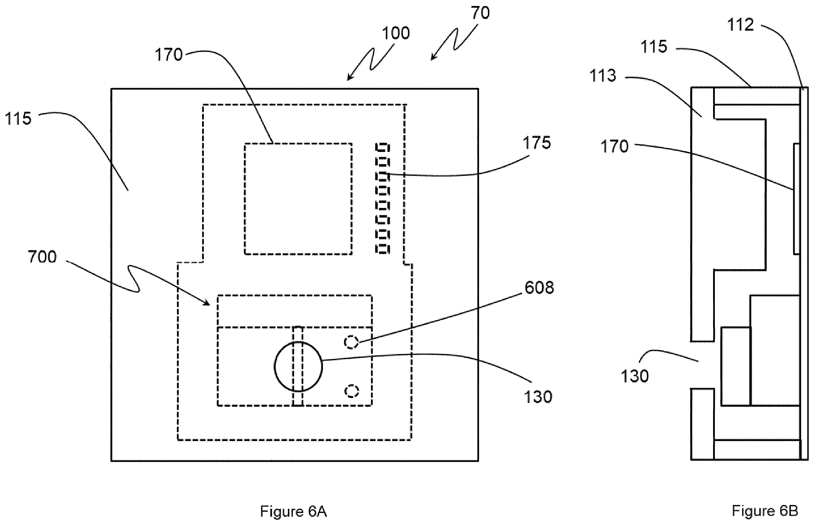

Figure 6A shows a top view of an embodiment of the environmental sensor assembly 70 of the present disclosure. The environmental sensor assembly 70 is shown with the package 100, so that the environmental sensor 700 and other components inside the package, are shown in broken lines, including the internal perimeter of the lateral or side walls of the package 100. The features of the environmental sensor located on the second surface of the sensor substrate are not shown for the sake of clarity.

As it can be seen from Figure 6A, the environmental sensor assembly 70 has an inlet 130 which is located on the lid 113 of the package 100, symmetrically with respect to the two regions of the environmental sensor 700. The environmental sensor 700 has a cap, of which a wall dividing the sensing region from the reference region is visible, and two holes 608 located on the cap, above the sensing region. The environmental sensor assembly 70 is further provided with an application-specific integrated circuit (ASIC) 170 and an electrical connection region 175. In this example, the lateral walls of the package have portions of different thickness so as to match the shape of the environmental sensor 700 and of the ASIC 170. In this way the internal dead volume of the package is minimized.

Figure 6B shows a side cross-section of the environmental sensor assembly 70. In this example, the lid 113 is shown having regions of different thickness opposite the environmental sensor 700 and the ASIC 170 to further reduce the package internal dead volume and matching, at least in part, the shape of the environmental sensor and the ASIC. In other words, the walls of the package may be described as defining a volume being, at least in part, of the same shape as the sensor and of the ASIC.

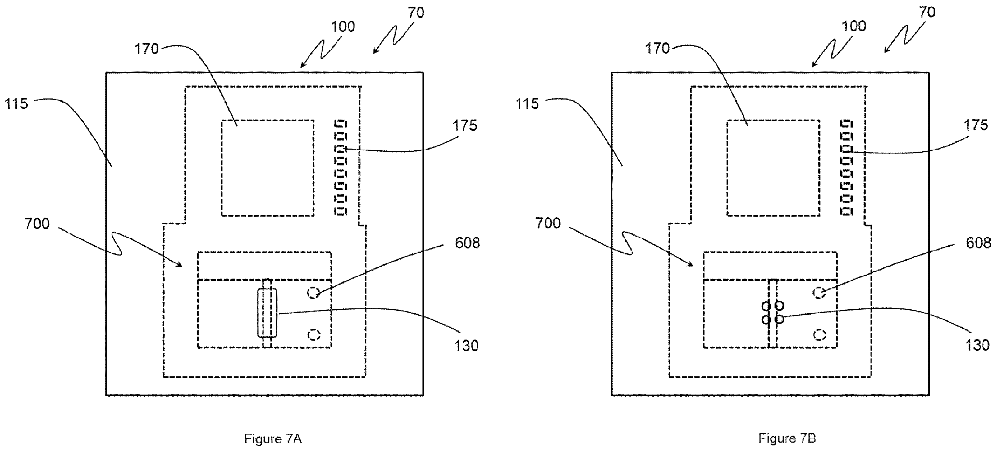

Figures 7A to 7D show further, non-exhaustive, examples of the environmental sensor assembly 70 comprising different arrangements of the inlet or inlets 130.

In Figure 7A, the package 100 has an inlet 130 which is elongated in one direction. The inlet is symmetrical with respect to the two regions of the environmental sensor 700.

In Figure 7B, the package 100 is provided with a plurality of inlets 130 which are arranged symmetrically with respect to the two regions of the environmental sensor 700.

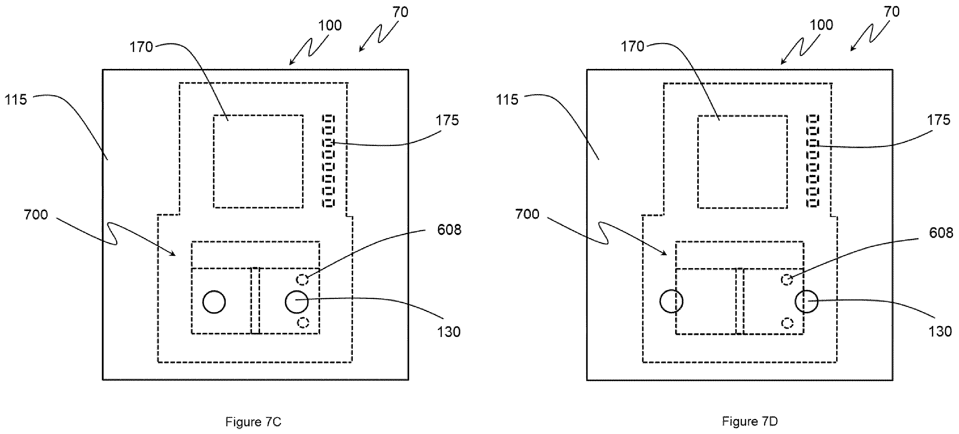

The packages of Figures 7C and 7D are provide with two inlets 130 which are arranged away from the center of the cap of the environmental sensor 700 but still symmetrical with respect to the two regions of the environmental sensor 700.

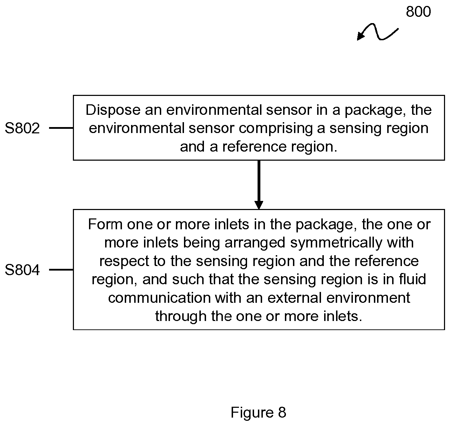

Figure 8 schematically illustrates an example of a method 800 of manufacturing an environmental sensor assembly according to the present disclosure. For example, the method 800 may be suitable for manufacturing any of the environmental sensor assemblies 10, 20, 30, 40, 50, 70 described and illustrated herein.

In a step S802, the method 800 comprises disposing an environmental sensor in a package, the environmental sensor comprising a sensing region and a reference region. It will be understood that the environmental sensor of the method 800 may be any suitable environmental sensor, such as any of the environmental sensors 200, 300, 400, 500, 600, 700 described and illustrated herein.

In a step S804, the method 800 comprises forming one or more inlets in the package, the one or more inlets being arranged symmetrically with respect to the sensing region and the reference region, and such that the sensing region is in fluid communication with an external environment through the one or more inlets. For example, the package of the method 800 may correspond to the package 100 described and illustrated herein. Forming the one or more inlets may comprise forming one or more openings in the package 100 by any suitable method, including drilling, or molding the package 100 such that the package 100 is formed with the one or more openings. Forming the one or more inlets according to the method 800 may result in a package 100 comprising one or more inlets 130 as described and illustrated herein.

It will be understood that the steps S802, S804 of the method 800 may be formed in any order. For example, the package could be received or produced, and the one or more inlets may be formed in the package before or after disposing the environmental sensor in the package. Alternatively, the package may be formed around the environmental sensor such that, once the package is formed, the environmental sensor is disposed in the package.

Although the disclosure has been described in terms of preferred embodiments as set forth above, it should be understood that these embodiments are illustrative only and that the claims are not limited to those embodiments. Those skilled in the art will be able to make modifications and alternatives in view of the disclosure which are contemplated as falling within the scope of the appended claims. Each feature disclosed or illustrated in the present specification may be incorporated in the disclosure, whether alone or in any appropriate combination with any other feature disclosed or illustrated herein.

## Claims

1. An environmental sensor assembly comprising:
a package; and
an environmental sensor disposed in the package, the environmental sensor comprising a sensing region and a reference region;
wherein the package comprises one or more inlets arranged symmetrically with respect to the sensing region and the reference region; and
wherein the sensing region is in fluid communication with an external environment through the one or more inlets.

2. The environmental sensor assembly according to claim 1, wherein the reference region is fluidically isolated from the external environment.

3. The environmental sensor assembly according to claim 1 or 2, wherein the reference region comprises a reference area fluidically isolated from the external environment.

4. The environmental sensor assembly according to any one of the preceding claims, wherein the environmental sensor comprises a cap defining a reference chamber, the reference region comprising the reference chamber, such that the reference chamber is fluidically isolated from the one or more inlets;
optionally wherein an inlet of the one or more inlets is located opposite a central region of the cap.

5. The environmental sensor assembly according to claim 4, wherein the cap further defines a sensing chamber;
wherein the sensing region comprises the sensing chamber; and
wherein the cap comprises one or more holes such that the sensing chamber is in fluid communication with the one or more inlets.

6. The environmental sensor assembly according to claim 5, wherein the one or more holes are laterally displaced with respect to the one or more inlets.

7. The environmental sensor assembly according to any one of the preceding claims, wherein the environmental sensor comprises:
a substrate comprising a sensing cavity within the sensing region and a reference cavity within the reference region;
a dielectric layer disposed over the substrate, wherein the sensing cavity defines a sensing membrane in the dielectric layer, and wherein the reference cavity defines a reference membrane in the dielectric layer; and
a first heating element on or within the sensing membrane, and a second heating element on or within the reference membrane.

8. The environmental sensor assembly according to claim 7 as dependent on claim 6 or claim 7, wherein the one or more holes are laterally displaced with respect to the sensing membrane.

9. The environmental sensor assembly according to any one of the preceding claims, comprising one or more inlets located on lateral walls of the package.

10. The environmental sensor assembly according to any one of the preceding claims, wherein a first inlet of the one or more inlets is located opposite the sensing region and a second inlet of the one or more inlets is located opposite the reference region.

11. The environmental sensor assembly according to any one of the preceding claims, comprising a circuit chip disposed inside the package;
optionally wherein the package comprises a package substrate, and wherein the circuit chip is disposed on a surface of the package substrate.

12. The environmental sensor assembly according to any one of the preceding claims, wherein the package comprises a first portion having a first thickness in a direction perpendicular to a surface of the package facing the sensor, and a second portion having a second thickness in a direction perpendicular to the surface of the package facing the sensor, wherein the first thickness is different from the second thickness.

13. The environmental sensor assembly according to any one of the preceding claims, wherein the package comprises a lid, a surface of the lid facing the sensor having a shape matching, at least in part, a shape of the sensor.

14. The environmental sensor assembly according to claim 11, wherein the package comprises a lid, a surface of the lid facing the sensor and the circuit chip having a shape matching, at least in part, a shape of the sensor and a shape of the circuit chip.

15. A method of manufacturing an environmental sensor assembly, the method comprising:
disposing an environmental sensor in a package, the environmental sensor comprising a sensing region and a reference region; and
forming one or more inlets in the package, the one or more inlets being arranged symmetrically with respect to the sensing region and the reference region, and such that the sensing region is in fluid communication with an external environment through the one or more inlets.
